# EUROPEAN PATENT SPECIFICATION

(11) **EP 1 769 763 B1**
(45) Date of publication and mention of the grant of the patent: **07.03.2018**
(21) Application number: 05025423.4
(22) Date of filing: 22.11.2005
(51) Int. Cl.: A61B 18/14, A61B 18/12

(54) **System for creating lesions using bipolar electrodes**
System zur Erzeugung von Läsionen unter Verwendung bipolarer Elektroden
Système pour la creation de lésions utilisant des électrodes bipolaires

(30) Priority: 30.09.2005 US 239999
(43) Date of publication of application: 04.04.2007
(73) Proprietor: Covidien AG, 8212 Neuhausen am Rheinfall (CH)
(72) Inventor: McPherson, James W., Boulder, CO 80301 (US)
(74) Representative: Soames, Candida Jane

(56) References cited:
- WO-A-99/22657
- DE-A1- 10 224 154
- FR-A- 2 864 439
- US-A1- 2002 193 851
- US-A1- 2004 181 216
- US-B1- 6 203 541
- US-B1- 6 506 189

## Description

### BACKGROUND

### 1. Technical Field

The present disclosure relates generally to a bipolar electrosurgical system as may be used for bipolar electrosurgery, and which may more particularly be applied to a system for creating lesions using bipolar electrodes.

### 2. Background of Related Art

Electrosurgery involves application of high frequency electrical current to a surgical site to cut, ablate, or coagulate tissue. In monopolar electrosurgery, a source or active electrode delivers radio frequency energy from the electrosurgical generator to the tissue and a return electrode carries the current back to the generator. In monopolar electrosurgery, the source electrode is typically part of the surgical instrument held by the surgeon and applied to the tissue to be treated. A patient return electrode is placed remotely from the active electrode to carry the current back to the generator.

In bipolar electrosurgery, one of the electrodes of the hand-held instrument functions as the active electrode and the other as the return electrode. The return electrode is placed in close proximity to the active (current supplying) electrode such that an electrical circuit is formed between the two electrodes (e.g., electrosurgical forceps). In this manner, the applied electrical current is limited to the body tissue positioned between the electrodes.

Bipolar electrosurgery has a number of advantages over monopolar electrosurgery. Bipolar electrosurgery generally requires lower power levels which results in less tissue destruction (e.g., tissue charring and scarring due to sparks at the electrodes). Bipolar electrosurgical techniques also reduce the danger of alternate site burns since no return electrodes are used and the only tissue destroyed is that located between the bipolar electrodes.

Bipolar electrosurgery is conventionally practiced using electrosurgical forceps-type device, where the active and return electrodes are housed within opposing forceps' jaws. Such bipolar electrosurgical devices use RF energy in conjunction with clamping force to coagulate vessels or tissue or seal blood vessels or tissue. Conventional bipolar electrosurgical devices are typically not adapted for creating lesions within organs due to their physical limitations.

Therefore there is a need for a system for creating lesions using bipolar electrosurgical devices.

DE 102 24 154 A discloses bipolar and multipolar electrosurgical devices having repectively two or more active electrodes, obviating the need for any patient return electrode. Electrical surgical energy is supplied to every active electrode.

### SUMMARY OF THE INVENTION

According to the present invention, there is provided a bipolar electrosurgical system as defined in appended independent claim 1. Preferred embodiments are described in the dependent claims.

### BRIEF DESCRIPTION OF THE DRAWINGS

To enable a better understanding of the present invention, and to show how the same may be carried into effect, reference will now be made, by way of example only, to the accompanying drawings in which:-
Fig. 1 is a schematic diagram of one embodiment of a bipolar electrosurgical system according to the present invention;
Fig. 2 is a diagram of an active and return ablation electrode pair;
Fig. 3 is a block and sectional diagram of the ablation electrode of Fig. 2;
Fig. 4 is a diagram of an ablation site having an active and return electrode;
Fig. 5 is a schematic block diagram illustrating automatic monitoring circuit;
Fig. 6 is a diagram of a resectioning procedure using the bipolar electrosurgical system of Fig. 1;
Fig. 7 is a flow chart illustrating a method for performing the resectioning procedure of Fig. 6;
Fig. 8 is a perspective view of an ablation device having a plurality of bipolar electrodes according to the present invention; and
Fig. 9 is a diagram of an ablation site having the ablation device of Fig. 8.

### DETAILED DESCRIPTION

The following description relates to a bipolar electrosurgical system. The system includes one or more elongated active and return electrode(s) configured to penetrate tissue to create one or more lesions having an ellipsoid-shaped cross section therein. The electrodes also include a thermal and electrical conducting rigid tubular member having a proximal and distal end with an insulative layer covering the external surface of the tubular member defining an exposed tip for conducting electrical energy therethrough.

According to one example to be described, a bipolar electrosurgical system is disclosed. The system includes at least one pair of active and return electrodes each including thermally-conductive tubular members with closed distal ends. Each of the tubular members include electrically conductive portions which are adapted to connect to an electrical energy source. The active and return electrodes are further configured to penetrate tissue and create at least one generally elliptical lesion therebetween upon activation of electrical energy. The system also includes a multiplexer disposed between the electrical energy source and each pair of electrically conductive active and return portions. The multiplexer is adapted to selectively switch electrical potentials of each pair of active and return electrically conductive portions to create lesions of varying geometry.

According to another described example, a method for performing an electrosurgical procedure is disclosed. The method includes the steps of providing at least one pair of active and return electrodes each including thermally-conductive tubular members with closed distal ends. Each of the tubular members includes electrically conductive portions which are adapted to connect to an electrical energy source. The active and return electrodes are configured to penetrate tissue and create at least one generally elliptical lesion therebetween upon activation of electrical energy. The method also includes the step of providing a multiplexer disposed between the electrical energy source and each pair of electrically conductive active and return portions. The multiplexer is adapted to selectively switch electrical potentials of each pair of active and return electrically conductive portions to create lesions of varying geometry.

Preferred embodiments of the present invention will be described hereinbelow with reference to the accompanying drawings. In the following description, well-known functions or constructions are not described in detail to avoid obscuring the present description with unnecessary detail.

Disclosed hereinbelow are a system and method for creating lesions using bipolar electrosurgical techniques and devices. The system includes at least one pair of electrodes, an active electrode and a corresponding return electrode. The electrodes are elongated electrodes configured to penetrate tissue and supply RF energy to the target site therein to create one or more lesions having a particularly-shaped cross section. For example, a plurality of electrode pairs may be utilized to create lesions which overlap to ablate a spherical/circular region of tissue (e.g., tumor) or a plurality of lesions may be created to ablate a strip of tissue to allow for bloodless resectioning of an organ.

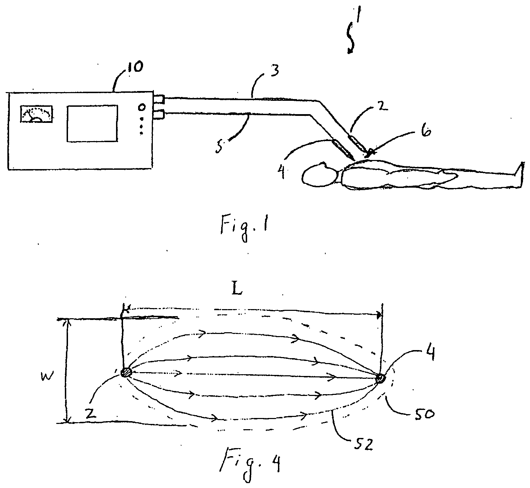

Fig. 1 is a schematic illustration of an electrosurgical system 1 according to the present invention. The system 1 includes an active electrode 2 and a return electrode 4 for treating tissue at a surgical site 6 of a patient. Electrosurgical energy is supplied to the active electrode 2 by a generator 10 via a cable 3 allowing the electrodes 2, 4 to ablate, cut or coagulate the tissue. The return electrode 4 is placed at the surgical site 6 to return the energy from the patient to the generator 10 via a cable 5.

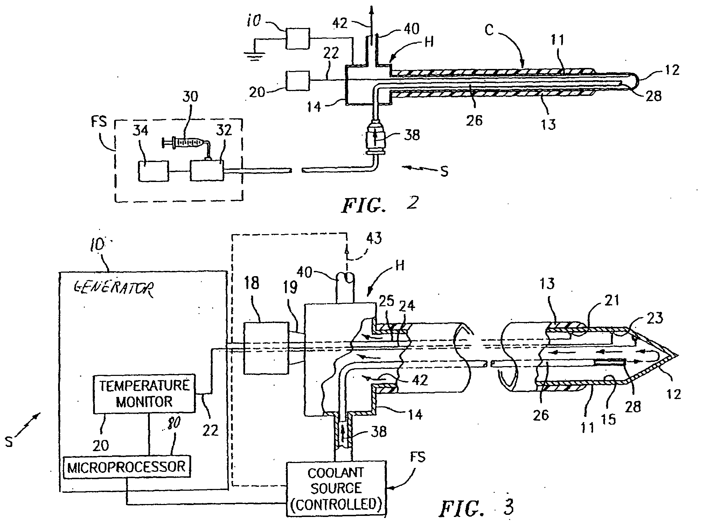

The active and return electrodes 2, 4 may be elongated electrodes configured to penetrate tissue and supply RF energy to the target site therein The active and return electrodes 2, 4 may also include a temperature control system, e.g., a coolant circulating system. Examples of an elongated electrode having a cooling system are shown and described in commonly-owned U.S. Patent Serial No. 6,506,189 entitled "Cool-tip electrode thermosurgery system" which is hereby referenced to the reader. However, a brief description of the relevant technology is provided below with reference to Figs. 2 and 3.

An elongated shaft or cannula body C is used for insertion of the active electrode 2 (or return electrode 4) either percutaneously or intraoperatively through an open wound site to the target site. As illustrated the cannula body C is integral with a head or hub element H coupled to remotely support components, collectively designated S.

As shown in Figs. 2 and 3, the cannula body C incorporates an elongated hollow ablative electrode 11 (e.g., active or return electrode 2, 4) formed of conductive material, (e.g. metal such as stainless steel, titanium, etc.). At the distal end of the cannula body C, the electrode 11 includes a shaft 15 which defines a tip 12 at a distal end thereof which may be of any shape or form (e.g., rounded or pointed). In one form, the tip 12 may define a trocar point and may be of robust metal construction to facilitate insertion or penetration of tissue. During an ablation procedure, the electrode 11 is inserted into the tissue and the generator 10 provides electrical current which spreads from the conductive portion, e.g. tip 12, to pass through the surrounding tissue thereby ablating the tissue and creating therapeutic lesions. Hence, when the tip 12 is positioned contiguous to tissue, energy from the generator 10 is dissipated into heat within the tissue.

As best shown in Fig. 3, electrode 11 includes an insulative coating 13 for preventing the flow of electrical current from the shaft 15 of electrode 11 into surrounding tissue. Thus, the insulative coating 13 shields the intervening tissue (i.e., tissue penetrated by the electrode 11 but not targeted for ablation) from RF current, so that such tissue is not substantially heated along the length of the shaft 15 except by the heating effect from the exposed portion or tip 12. It should be appreciated that the length of the exposed portion or tip 12 is directly related to the size of the lesion created (i.e., the larger the exposed portion of the electrode 11 the larger is the lesion).

At its proximal end, the electrode 11 is typically integrally associated with an enlarged housing 14 of the hub H which carries electrical and coolant connections as explained in greater detail below. Outside the patient's body, the housing 14 defines ports for connections to the support components S (e.g., electrical and fluid couplings). As suggested, the housing 14 may be integral with the electrode 11, formed of metal, or it may constitute a separate subassembly as described below. Alternatively, the housing 14 can be made of plastic, accommodating separate electrical connections. In that regard, a plastic housing 14 is preferred, due to low artifact imaging it exhibits in various imaging techniques (e.g., X-ray, CT, MRI, etc.)

Referring to Fig. 2, the housing 14 mates with a block 18 thereby defining a luer taper lock 19 which seals the block 18 and the housing 14. In addition, fluid and electrical couplings are provided. Specifically, connection to the generator 10 (e.g., the cables 3, 5 of Fig. 1) may be a standard cable connector, a leader wire, a jack-type contact or other connector designs known in the art. The temperature-sensing and radiofrequency electrical connections can be made through the housing 14 and extend to the region of the tip 12, where an RF line 25 is connected by junction 21 (e.g., a weld, braze, or other secure electrical connection). Sensor line 24 extends to a temperature sensor 23 (a thermistor, a thermocouple, or other type of sensor) which may be fused or in thermal contact with the wall of the tip 12 to sense temperature condition at or proximate of the tip 12.

The generator 10 may be connected to reference potential and coupled through the block 18 affixed to the hub H. Specifically, the generator 10 provides RF voltage through the block 18 with an electrical connection to the electrode 11 as indicated by the line 25 (e.g., the cables 3, 5), to the connection junction 21. The generator 10 may take the form of an RF generator as exemplified by the RFG-3C RF Lesion Generator System available from Radionics, Inc. of Burlington, Massachusetts.

The ablation electrode 11 includes a number of systems for regulating the temperature generated at the ablation site. One such system utilizes cooling fluid injected into the ablation electrode 11 based on temperature readings. In that regard, a temperature monitor 20 is electrically connected by lines 22 and 24 to a temperature sensor 23 as in the form of a thermocouple or thermistor typically within or contacting the tip 12. As illustrated, the temperature sensor 23 is connected to the tip 12. The sensed temperature is utilized to control either or both of the flow of RF energy or the flow of coolant to attain the desired ablation while maintaining the maximum temperature substantially below 100 °C or another threshold temperature. A plurality of sensors may be utilized including units extending outside the tip 12 to measure temperatures existing at various locations in the proximity of the tip 12. The temperature monitor 20 may be as exemplified by the TC thermocouple temperature monitoring devices available from Radionics, Inc. of Burlington, Massachusetts.

Temperatures at, or near the tip 12 may be controlled by controlling the flow of fluid coolant through the ablation electrode 11. Accordingly, the temperature of the tissue contacting at or near the tip 12 is controlled. In the disclosed embodiment, fluid from a fluid source FS is carried the length of the ablation electrode 11 through a tube 26 extending from the housing (hub) H to the distal end of the electrode 11 terminating in an open end 28 at the tip 12. At the opposite end of the electrode 11, within the housing (hub) H, the tube 26 is connected to receive fluid. As illustrated in the detailed structure of Figs. 2 and 3, the fluid source FS includes a source unit 34 coupled through a control 32 utilizing a hypodermic syringe 30 (or other fluid delivery mechanism) to actuate fluid flow, as represented by an arrow, through a coupling 38. Thus, fluid flow is regulated in accordance with observed temperature, allowing increased flow of RF energy.

The fluid coolant may take the form of water or saline solution which is typically used for heat dissipation via convectional removal of heat from the tip 12. The reservoir or source unit 34 might be a large reservoir of cooled water, saline or other fluid. As a simplistic example, a tank of water with ice cubes can function to maintain the coolant at a temperature of approximately 0°C. As another example, the fluid source FS could incorporate a peristaltic pump or other fluid pump, or could merely be a gravity feed for supplying fluid from a flexible bag or rigid tank.

Backflow from the tip 12 is through an exit port 40 of the hub H as illustrated by arrows 42, 43. The port 40 may be in the form of simple couplings, rigid units or may comprise flexible
tubular couplings to reduce torque transmission to the electrode 11. Also, the coolant flow members may simply take the form of PVC tubes with plastic luer connectors for ease of use.

As a result of the coolant flow, the interior of the electrode 11, more specifically the electrode tip 12, can be held to a temperature near that of the fluid source FS. The coolant can circulate in a closed system as illustrated in Fig. 2. Also, in some situations, it may be desirable to reverse the direction of fluid flow from that depicted in the figures. As treated in detail below, coordinated operation, involving RF heating along with the cooling may be accomplished by a microprocessor 80, which is coupled to the generator 10, the temperature monitor 20 and the fluid source FS to receive data on flow rates and temperatures and exercise control. Accordingly, an integrated operation is provided with feedback from the temperature monitor 20 in a controlled format and various functions can be concurrently accomplished. Thus, facilitated by the cooling, the ablation electrode 11 is moderated, changed, controlled or stabilized. Such controlled operation can effectively reduce the temperature of tissue near the tip 12 to accomplish an equilibrium temperature distribution tailored to the desired size of the desired lesion.

The temperature distribution in the tissue near the tip 12 depends on the RF current from the tip 12 and depends on the temperature of the tissue which is adjacent to the tip 12. Tip temperature can be controlled by the flow of fluid from the source FS. Thus, a thermal boundary condition is established, holding the temperature of the tissue (near the tip 12) to approximately the temperature of the tip itself, e.g. the temperature of the fluid inside the tip 12. Accordingly, by temperature control, a surgeon may impose a defined temperature at the boundary of the electrode tip 12 which can be somewhat independent of the RF heating process, and in fact, dramatically modify the temperature distribution in the tissue.

During a bipolar electrosurgical procedure according to the present disclosure, active and return electrodes 2, 4 (Fig. 1) are placed at the surgical site 6 in such a way as to create a lesion 50 as shown in Fig. 4. The current travels through tissue from the active electrode 2 to the return electrode 4 as represented by the current flow 52. Due to the current flow 52 forming a generally elliptical pattern, the resulting lesion 50 also has an elliptical shape with a length L (e.g., major axis), a width W (e.g., minor axis), and depth D (not shown). Those skilled in the art will appreciate that the depth D is directly proportional to the length of the exposed conductive tip of the active and return electrodes 2, 4 (or electrode 11 of Figs 2 and 3).

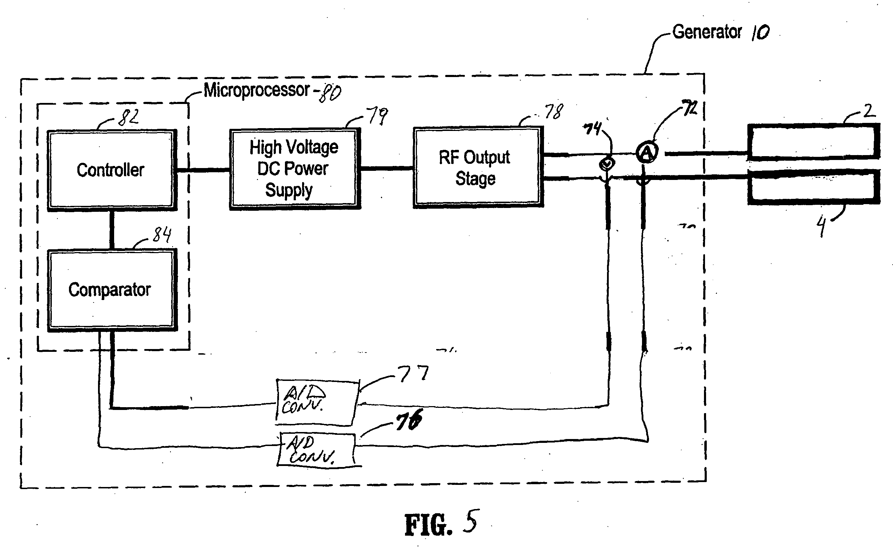

It is also envisioned that impedance of the tissue between the active and return electrodes 2, 4 is monitored to allow the user to selectively regulate the current applied to the tissue to obtain a desired volumetric measure of the lesion 50. For example, an impedance reading above a predetermined threshold would signal the generator 10 to shut down, thereby terminating the current flow once the lesion 50 reaches the desired volume. One example of a bipolar system having a generator controlled by an impedance sensor is shown and described in commonly-owned U.S. Patent Serial No. 6,203,541 entitled "Automatic Activation of Electrosurgical Generator Bipolar Output" which is hereby referenced to the reader. However, a brief description of the relevant technology is provided below with reference to Fig. 5.

Fig. 5 shows a schematic diagram of the bipolar electrosurgical system of the present disclosure. As the impedance of the tissue changes the current changes inversely proportionally if the voltage remains constant. This is defined by Ohm's Law: V = RI, wherein V is the voltage across the electrodes in volts, I is the current through the electrodes (and tissue) in milliamps and R is the resistance or impedance of the tissue measured in Ohms. By this equation it can be readily appreciated that when the tissue impedance increases, the current will decrease and conversely, if the tissue impedance decreases, the current will increase. The electrosurgical system of the present disclosure essentially measures impedance based on the changes in current. Prior to electrosurgical treatment, tissue is more conductive, so when energy is applied, the impedance is relatively low. As the tissue is treated and a lesion is created, the conductivity decreases as the tissue moisture content decreases and consequently tissue impedance increases.

The active and return electrodes 2, 4 are connected to the generator 10. The electrosurgical generator 10 includes a current sensor 72 electrically connected to the active electrode 2 and a voltage sensor 74 electrically connected between the active and return electrodes 2, 4. The current sensor 72 measures the current and the voltage sensor 74 detects the voltage between the active and return electrodes 2, 4 at the target tissue. The current and voltage sensors 72, 74 feed analog voltage and current signals to analog to digital converters 76, 77 respectively.

The analog to digital converters 76, 77 receive the analog signals and convert it to a digital signal for transmission to the microprocessor 80, which preferably includes a comparator 84 and a controller 82. An output port of the microprocessor 80 is electrically connected to a high voltage DC power supply 79. The microprocessor 80 calculates the impedance according to Ohm's Law.

The comparator 84 evaluates the digital impedance signal by comparing it to predetermined impedance values and generates responsive signals for transmission to the controller 82 as described in detail below. In response to the signals received from the comparator 84, the controller 82 generates and transmits control signals to the power supply 79 which in turn controls the energy output of the RF output stage 78 which delivers current to the active and return electrodes 2, 4.

The deactivation threshold value is preferably about 2000 Ohms or another threshold (e.g., tissue determined baseline). If the impedance calculation exceeds the deactivation threshold, this indicates that the tissue has been treated since the impedance increases as the tissue is ablated because its conductivity due to moisture loss has decreased. If the deactivation threshold is exceeded, a digital deactivation signal is transmitted from the comparator 84 to the controller 82 (Fig. 5A). Thereafter the controller 82 signals the power supply 79 to automatically deactivate the generator so current output from the RF output stage 78 is terminated, thereby preventing overheating and unwanted destruction of tissue. This system provides for automatic deactivation of the generator 10 based on impedance measurements as soon as the lesion is complete.

Lesions are generally used in electrosurgical procedures where a specific region of the tissue must be destroyed (e.g., a tumor). More specifically, conventional lesions are generally spherical (e.g., circular cross section) since this shape allows for optimum coverage of the target area. Sperical lesions are generally formed using monopolar electrosurgery. During monopolar electrosurgical procedures, current travels outward from an active electrode placed at the center of the tissue throughout the target area resulting in a lesion having a spherical shape.

Although spherical lesions are useful in ablating regions of tissue due to its optimum area of effect, in certain procedures it is preferred to create lesions of an elongated shape, such as the ellipsoid shape of the lesion 50 (Fig. 4). The elongated ellipsoid shape of the lesion 50 allows for tissue ablation in a narrow area (e.g., a strip) while preserving more of the surrounding tissue. This shape is particularly useful in bloodless resectioning procedures performed on organs containing large amount of blood vessels (e.g., liver) where removal of a section of the organ requires electrosurgically treating the multitude of blood vessels present therein.

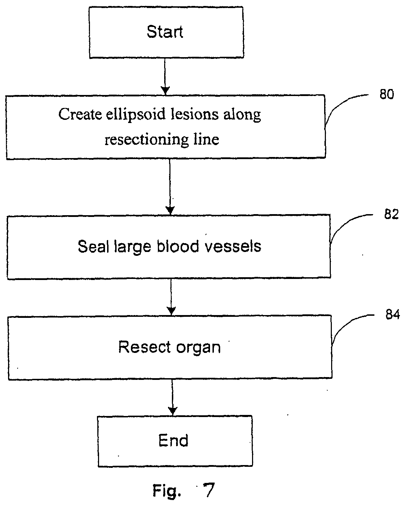

More particularly and with reference to Figs. 6 and 7, a liver 54 is shown which is to be resectioned, such that a resectioned portion 55 will be detached from the liver 54 along a resectioning line 56. In step 80, a plurality of lesions 50 are created along the resectioning line 56 by inserting the active and return electrodes 2, 4 therein separated by a predetermined length L. The lesions 50 are created so that the major axis thereof is along the resectioning line 56 and the lesions 50 are connected end to end (e.g., insertion points of active and return electrodes 2, 4) with slight overlap of the edges.

The length L of the lesion 56 is selected by the surgeon depending on the desired shape and size. The size of the length L is inversely proportional to the width W, thus increasing the length L, decreases the width W. However, the separation between the active and return electrodes 2, 4 (e.g., length L) also depends on the amount of energy supplied to the active electrode 2. The lesion 50 having a relatively short length L requires less energy to form, while the lesion 50 with a longer length L requires more power. Therefore, the surgeon has to determine the optimum length L of the lesions 50 based on the desired size, shape, and amount of current prior to creating the lesions 50. In addition, the depth D of the lesion 50 is equivalent to the length of the exposed tip 12. Thus, by adjusting the insulation (e.g., the insulative coating 13) covering the active and return electrodes 2, 4 the surgeon controls the depth D of the lesion 50.

Once the lesions 50 have been created, small blood vessels (e.g., capillaries) are treated to reduce/stop blood flow. This allows organs of high vascularity to be resectioned without major blood loss. However, large blood vessels are not sealed during tissue ablation, as performed in step 80. Therefore, in step 82, the larger blood vessels are sealed. This may be performed in a plurality of ways. For instance, conventional sealing techniques using mechanical pressure and/or radio frequency energy may be used to create effective seals. One example of treating tissue is by sealing the tissue or vessels to stop bleeding. Sealing is defined as a process which precisely controls closure pressure, distance between the electrodes (i.e., gap distance), energy parameters to fuse opposing tissue structures into a homogenous mass with limited demarcation between tissue structures. Examples of vessel sealing devices are shown in commonly owned U.S. Application Serial No. 10/460,926 published as US2004254573 entitled "Vessel sealer and divider for use with small trocars and cannulas," U.S. Application Serial No. 10/953,757 Published as US2005107785 entitled "Vessel sealer and divider having elongated knife stroke and safety for cutting mechanism," U.S. Application Serial No. 10/873,860 published as US2005107784 "Open vessel sealing instrument with cutting mechanism and distal lockout," U.S. Application Serial No. 10/991,157 published as US2005119655 entitled "Open vessel sealing instrument with cutting mechanism," and U.S. Application Serial No. 10/962,116 published as US2005154387 "Open vessel sealing instrument with hourglass cutting mechanism and over-ratchet safety," the contents of all of which are hereby referenced to the reader.

Once the large blood vessels are sealed, in step 82, the liver 54 is resected along the resectioning line 56 to separate the resectioned portion 55. A plurality of cutting apparatus may be used, such as conventional scalpels and/or electrosurgical cutting devices.

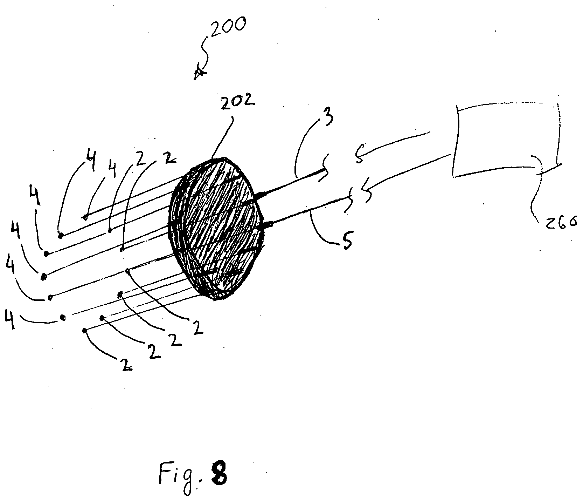

Although bipolar systems provide a number of advantages discussed above (e.g., smaller energy requirement, lack of return electrode pads, lack of off-site bums, etc.) the particular lesion 50 created using the bipolar electrosurgical system and method of Figs. 4-7 is not well suited for performing other ablation procedures due to the resulting ellipsoid shape. Therefore, it is envisioned that the presently-described bipolar electrosurgical system may also be configured to create spherical lesions as shown in Fig. 8.

For example, Fig. 8 shows an ablation device 200 having six pairs of bipolar electrodes (e.g., the active and return electrodes 2, 4) arranged in a generally circular pattern. Those skilled in the art will appreciate that the number of electrodes in the ablation device 200 depends on a number of factors (e.g., size of the lesion, power level, etc.). The electrodes are held by a housing 202 which contains the cables 3, 5 providing an electrical connection to the generator 10. It is envisioned that the housing 202 may have an adjustable circumference thereby allowing the lesion area to be ablated by the electrodes to be regulated according to a specific purpose.

The ablation device 200 allows for creation of a lesion 64 which more closely approximates a circle by using a series of pairs of bipolar electrodes (e.g., the active and return electrodes 2, 4) arranged in a circular pattern as shown. As can be appreciated, the lesion 64 is better suited for covering a circular/spherical target area in need of ablation (e.g., a tumor 60). Lesion 64 is created by multiplexing the RF energy in different directions which involves switching the RF energy through each pair of the active and return electrodes 2, 4 as indicated by the arrows 62 representing the current flow. This would be accomplished by passing electrical energy sequentially through the active electrode(s) 2 while including only the corresponding return electrode(s) 4 in the circuit so that the current flows in one particular direction. It is envisioned a multiplexer 260 may be employed to control switching of the active and return electrodes 2, 4. For example, it is envisioned that multiplexer 260 may be configured to regulate the current in any fashion by switching on and off various pairs of active and return electrode pairs to create lesions 50. Moreover it is also contemplated that multiplexer 260 may be configured to change active and return electrode to reverse polarity and reverse the current therethrough the lesions 50 depending on particular purpose.

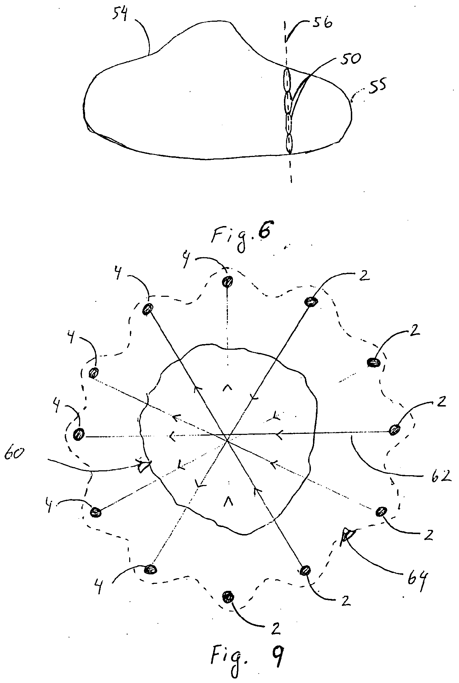

With respect to Fig. 8 and as a result of multiplexing, each pair of the active and return electrodes 2, 4 generates a lesion having an ellipsoid shape. A plurality of the ellipsoid lesions having the same center overlap and form the lesion 64, which closely approximates a sperical lesion which has been conventionally created using monopolar devices. Those skilled in the art will appreciate that Fig. 9 shows only a cross section of the lesion 64 and that the lesion 64 has a depth equivalent to the exposed tips of the active and return electrodes 2, 4.

## Claims

1. A bipolar electrosurgical system comprising:
a plurality of pairs of an active (2) and a return (4) electrode each including thermally-conductive tubular members (11) with closed distal ends (12), the tubular members each including a respective active or return electrically conductive portion which is adapted to connect to an electrical energy source (10) connectable to reference potential, the active (2) and return (4) electrodes configured to penetrate tissue (6, 60) and create at least one generally elliptical lesion (50, 64) therebetween upon activation of electrical energy; and
a multiplexer (266) disposed between the portions adapted to connect to the electrical energy source (10) and each pair of electrically conductive active and return portions, said multiplexer adapted to supply electrical energy to the active electrode (2) from the electrical energy source (10), to return the supplied electrical energy to the electrical energy source (10) from the penetrated tissue (6, 60) by the return electrode (4), and to selectively switch the electrical potential of each pair of active and return electrically conductive portions to create lesions (50, 54) of varying geometry, **characterized in that** as a result of multiplexing by the multiplexer, each pair of the active and return electrodes (2, 4) generates a lesion having an ellipsoid shape and a plurality of the ellipsoid shaped lesions having the same center overlap.

2. A bipolar electrosurgical system of claim 1, further comprising:
a current sensor (72) configured to measure current between each pair of active (2) and return (4) electrodes; and
a voltage sensor (74) configured to measure voltage between each pair of active (2) and return (4) electrodes.

3. A bipolar electrosurgical system of claim 2, further comprising:
a microprocessor (80) in electrical communication with the current sensor (72) configured to calculate the impedance between the active electrode (2) and the return electrode (4) based on the measured current and measured voltage;
a comparator (34) operatively associated with the electrical energy source (10) and configured to compare the calculated impedance to an activation range of impedance values; and
a controller (2) operatively associated with the electrical energy source (10) and configured to automatically deactivate the electrical energy source (10) if the calculated impedance exceeds a deactivation threshold.

4. A bipolar electrosurgical system of claim 3, wherein the deactivation threshold is 2000 Ohms.

5. A bipolar electrosurgical system of claim 3 or 4, further comprising a filter for blocking energy from an output of the electrical energy source (10) from the impedance detection circuit, the filter being in electrical communication with the current sensor (72).

6. A bipolar electrosurgical system of any one of the preceding claims, wherein at least each active electrode (2) of said at least one pair of active (2) and return (4) electrode further comprises:
a first interior cavity extending from the closed distal end (12) of the tubular member (11) to a proximal end thereof;
a first fluid conduit (26) sized to extend into the first interior cavity and adapted to be connected to a source of coolant (34) to supply coolant for cooling tissue contiguous to the first exposed portion;
a temperature sensor (23) disposed within the first interior cavity configured to detect a temperature; and
a regulator (32) operatively connected to the source of coolant (34) configured to adaptively provide coolant to the fluid conduit (26) according to the measured temperature.

## Patentansprüche

1. Bipolares elektrochirurgisches System, umfassend:
eine Vielzahl von Paaren einer aktiven Elektrode (2) und einer Rückleitelektrode (4), von denen jede wärmeleitfähige, röhrenförmige Elemente (11) mit geschlossenen distalen Enden (12) enthält, wobei alle röhrenförmige Elemente einen jeweiligen elektrisch leitfähigen aktiven oder Rückleitabschnitt enthalten, der angepasst ist, um an eine elektrische Energiequelle (10) angeschlossen zu werden, die an Bezugspotenzial anschließbar ist, wobei die aktive (2) und die Rückleitelektrode (4) konfiguriert sind, Gewebe (6, 60) zu durchdringen und mindestens eine allgemein elliptische Läsion (50, 64) dazwischen nach Aktivierung von elektrischer Energie zu erzeugen; und
einen Multiplexer (266), der zwischen den Abschnitten angeordnet ist, angepasst, um an die elektrische Energiequelle (10) und jedes Paar elektrisch leitfähiger aktiver und Rückleitabschnitte angeschlossen zu werden, wobei der Multiplexer angepasst ist, um elektrische Energie an die aktive Elektrode (2) aus der elektrischen Energiequelle (10) zu liefern, die gelieferte elektrische Energie an die elektrische Energiequelle (10) von dem durchdrungenen Gewebe (6, 60) durch die Rückleitelektrode (4) zurückzuleiten und selektiv das elektrische Potenzial von jedem Paar eines elektrisch leitfähigen aktiven und Rückleitabschnitts umzuschalten, um Läsionen (50, 54) von variierender Geometrie zu erzeugen,
**dadurch gekennzeichnet, dass** als ein Ergebnis des Multiplexens durch den Multiplexer jedes Paar aktiver und Rückleitelektrode (2, 4) eine Läsion erzeugt, die eine ellipsoidische Form aufweist, und eine Vielzahl der ellipsoidisch geformten Läsionen, welche dieselbe Zentrumüberlappung aufweisen.

2. Bipolares elektrochirurgisches System nach Anspruch 1, weiter umfassend:
einen Stromsensor (72), der konfiguriert ist, um Strom zwischen jedem Paar aktiver Elektrode (2) und Rückleitelektrode (4) zu messen; und
einen Spannungssensor (74), der konfiguriert ist, um Spannung zwischen jedem Paar aktiver Elektrode (2) und Rückleitelektrode (4) zu messen.

3. Bipolares elektrochirurgisches System nach Anspruch 2, weiter umfassend:
einen Mikroprozessor (80) in elektrischer Verbindung mit dem Stromsensor (72), der konfiguriert ist, um die Impedanz zwischen der aktiven Elektrode (2) und der Rückleitelektrode (4) auf der Basis des gemessenen Stroms und der gemessenen Spannung zu errechnen;
einen Vergleicher (34), der operativ mit der elektrischen Energiequelle (10) assoziiert ist und konfiguriert ist, um die errechnete Impedanz mit einem Aktivierungsbereich von Impedanzwerten zu vergleichen; und
einen Regler (2), der operativ mit der elektrischen Energiequelle (10) assoziiert ist und konfiguriert ist, um automatisch die elektrische Energiequelle (10) zu deaktivieren, wenn die errechnete Impedanz eine Deaktivierungsschwelle übersteigt.

4. Bipolares elektrochirurgisches System nach Anspruch 3, wobei die Deaktivierungsschwelle 2000 Ohm beträgt.

5. Bipolares elektrochirurgisches System nach Anspruch 3 oder 4, weiter umfassend einen Filter zum Blockieren von Energie aus einer Abgabe der elektrischen Energiequelle (10) von der Impedanzerkennungsschaltung, wobei der Filter in elektrischer Verbindung mit dem Stromsensor (72) steht.

6. Bipolares elektrochirurgisches System nach einem der vorhergehenden Ansprüche, wobei mindestens jede aktive Elektrode (2) des mindestens einen Paars aktiver Elektrode (2) und Rückleitelektrode (4) weiter umfasst:
einen ersten inneren Hohlraum, der sich von dem geschlossenen distalen Ende (12) des röhrenförmigen Elements (11) zu einem proximalen Ende davon erstreckt;
eine erste Flüssigkeitsleitung (26), so dimensioniert, dass sie sich in den ersten inneren Hohlraum erstreckt und angepasst, um an eine Kühlmittelquelle (34) angeschlossen zu werden und Kühlmittel zum Kühlen von Gewebe bereitzustellen, das an den ersten freigelegten Abschnitt angrenzt;
einen Temperatursensor (23), der im Inneren des ersten inneren Hohlraums angeordnet ist, der konfiguriert ist, eine Temperatur festzustellen; und
einen Regler (32), der operativ an die Kühlmittelquelle (34) angeschlossen ist und konfiguriert ist, adaptiv Kühlmittel an die Flüssigkeitsleitung (26) entsprechend der gemessenen Temperatur zu liefern.

## Revendications

1. Système électrochirurgical bipolaire comprenant :
une pluralité de paires d'une électrode active (2) et d'une électrode de retour (4) comprenant chacune des éléments tubulaires thermoconducteurs (11) avec des extrémités distales fermées (12), les éléments tubulaires comprenant chacun une partie électroconductrice active ou de retour respective qui est adaptée pour se connecter à une source d'énergie électrique (10) qui peut être connectée à un potentiel de référence, les électrodes active (2) et de retour (4) étant configurées pour pénétrer dans un tissu (6, 60) et créer au moins une lésion généralement elliptique (50, 64) entre elles lors de l'activation de l'énergie électrique ; et
un multiplexeur (266) disposé entre les parties adaptées pour se connecter à la source d'énergie électrique (10) et à chaque paire de parties active et de retour électroconductrices, ledit multiplexeur étant adapté pour fournir de l'énergie électrique à l'électrode active (2) à partir de la source d'énergie électrique (10) pour renvoyer l'énergie électrique fournie à la source d'énergie électrique (10) depuis le tissu pénétré (6, 60) par l'électrode de retour (4) et commuter sélectivement le potentiel électrique de chaque paire de parties électroconductrices active et de retour afin de créer des lésions (50, 54) de géométrie variable,
**caractérisé en ce qu'**à la suite du multiplexage par le multiplexeur, chaque paire des électrodes active et de retour (2, 4) génère une lésion ayant une forme ellipsoïdale et une pluralité des lésions de forme ellipsoïdale ayant le même chevauchement central.

2. Système électrochirurgical bipolaire selon la revendication 1, comprenant en outre :
un capteur de courant (72) configuré pour mesurer le courant entre chaque paire d'électrodes active (2) et de retour (4) ; et
un capteur de tension (74) configuré pour mesurer la tension entre chaque paire d'électrodes active (2) et de retour (4).

3. Système électrochirurgical bipolaire selon la revendication 2, comprenant en outre :
un microprocesseur (80) en communication électrique avec le capteur de courant (72) configuré pour calculer l'impédance entre l'électrode active (2) et l'électrode de retour (4) sur la base du courant mesuré et de la tension mesurée ;
un comparateur (34) associé en service à la source d'énergie électrique (10) et configuré pour comparer l'impédance calculée à une plage d'activation de valeurs d'impédance ; et
un dispositif de commande (2) associé en service à la source d'énergie électrique (10) et configuré pour désactiver automatiquement la source d'énergie électrique (10) si l'impédance calculée dépasse un seuil de désactivation.

4. Système électrochirurgical bipolaire selon la revendication 3, dans lequel le seuil de désactivation est de 2 000 ohms.

5. Système électrochirurgical bipolaire selon la revendication 3 ou 4, comprenant en outre un filtre pour bloquer l'énergie venant d'une sortie de la source d'énergie électrique (10) du circuit de détection d'impédance, le filtre étant en communication électrique avec le capteur de courant (72).

6. Système électrochirurgical bipolaire selon l'une quelconque de revendications précédentes, dans lequel au moins chaque électrode active (2) de ladite au moins une paire d'électrodes active (2) et de retour (4) comprend en outre :
une première cavité interne s'étendant de l'extrémité distale fermée (12) de l'élément tubulaire (11) à son extrémité proximale ;
un premier conduit de fluide (26) calibré pour s'étendre dans la première cavité interne et adapté pour être connecté à une source de réfrigérant (34) pour fournir un réfrigérant pour refroidir le tissu contigu à la première partie exposée ;
un capteur de température (23) disposé dans la première cavité interne configuré pour détecter une température ; et
un régulateur (32) connecté en service à la source de réfrigérant (34) configuré pour fournir de manière adaptative du réfrigérant au premier conduit (26) selon la température mesurée.
